# EUROPÄISCHE PATENTANMELDUNG

(11) **EP 3 699 546 A1**
(43) Veröffentlichungstag der Anmeldung: **26.08.2020**
(21) Anmeldenummer: 20155072.0
(22) Anmeldetag: 03.02.2020
(51) Int. Cl.: G01B 11/02, G01B 11/28, G01B 11/06, G01B 11/24, G01B 11/08, G01N 11/02, G01N 33/38

(54) **VERFAHREN UND ANORDNUNG ZUR ERMITTLUNG MINDESTENS EINER KENNGRÖSSE VON FRISCHBETON**

(30) Priorität: 23.02.2019 DE 102019001313
(71) Anmelder: Züblin Spezialtiefbau GmbH, 70567 Stuttgart (DE)
(72) Erfinder: Bellato, Diego, 73760 Ostfildern (DE)
(74) Vertreter: Reinhardt, Annette

(57) **Zusammenfassung**

Ein Verfahren zur Ermittlung von mindestens einer Kenngröße von Frischbeton (6), bei dem eine Probenmenge des Frischbetons (6) auf eine Platte (1) aufgebracht wird, umfasst die optische Erfassung mindestens eines Parameters des Frischbetons (6) mittels einer optischen Erfassungseinheit (7), die automatisierte Bestimmung mindestens einer Kenngröße des Frischbetons (6) aus dem mindestens einen Parameter und die Speicherung der mindestens einen Kenngröße in einem Datenspeicher (24). Eine Anordnung zur Ermittlung mindestens einer Kenngröße von Frischbeton (6) umfasst eine Platte (1), mindestens eine optische Erfassungseinheit (7) sowie einen Datenspeicher (24).

## Beschreibung

Die Erfindung betrifft ein Verfahren und eine Anordnung zur Ermittlung mindestens einer Kenngröße von Frischbeton.

Zur Bestimmung von Kenngrößen von Frischbeton sind unterschiedliche Verfahren bekannt, die vom Arbeiter auf der Baustelle auszuführen sind. Zur Bestimmung der Verarbeitbarkeit des Frischbetons wird nach normierter Aufbringung einer Probenmenge von Frischbeton auf eine Platte der Durchmesser des sich ergebenden Betonkuchens in zwei Richtungen gemessen. Aus diesen Maßen wird das Ausbreitmaß berechnet und vom Arbeiter notiert. Bei Bestimmung der visuellen Stabilität des Betons erfolgt üblicherweise eine Einteilung in eine von mehreren Stabilitätsklassen. Hierzu muss der Arbeiter den Betonkuchen optisch bewerten. Dieses Verfahren ist sehr subjektiv. Die Ermittlung der Kenngrößen ist außerdem vergleichsweise zeitaufwändig.

Der Erfindung liegt die Aufgabe zugrunde, ein Verfahren zur Ermittlung mindestens einer Kenngröße von Frischbeton anzugeben, das bei verringertem Zeitaufwand bessere, objektivere Ergebnisse liefert. Eine weitere Aufgabe der Erfindung besteht darin, eine Anordnung zur Ermittlung mindestens einer Kenngröße von Frischbeton anzugeben, mit der die Ermittlung mindestens einer Kenngröße des Frischbetons vereinfacht ist.

Diese Aufgabe wird bezüglich des Verfahrens durch ein Verfahren mit den Merkmalen des Anspruchs 1 gelöst. Bezüglich der Anordnung wird die Aufgabe durch eine Anordnung mit den Merkmalen des Anspruchs 11 gelöst.

Für das Verfahren zur Ermittlung mindestens einer Kenngröße von Frischbeton ist vorgesehen, bei einer auf eine Platte aufgebrachten Probemenge von Frischbeton mindestens einen Parameter des Frischbetons mittels einer optischen Erfassungseinheit optisch zu erfassen, automatisiert mindestens eine Kenngröße des Frischbetons aus dem mindestens einen Parameter zu bestimmen und die mindestens eine Kenngröße in einem Datenspeicher zu speichern. Die optische Erfassung, Bestimmung der Kenngröße und Abspeicherung der Kenngröße erfolgt automatisiert ohne Zutun eines Bedieners. Der Arbeiter muss daher für das Verfahren lediglich die Probenmenge des Frischbetons in bekannter Weise auf die Platte aufbringen.

Parameter der Probemenge des Betons können insbesondere Abmessungen oder Flächen der Probenmenge auf der Platte sein. Die Abmessungen können dabei als maximale Abmessung in einer oder mehreren Richtungen ermittelt werden oder als ortsbezogene Abmessungen, insbesondere als zweidimensionales oder dreidimensionales Höhenprofil, ermittelt werden.

Durch die optische Erfassung des mindestens einen Parameters des Frischbetons, beispielsweise eines Maßes der Probenmenge des Frischbetons, kann auf einfache Weise eine höhere Genauigkeit bei der Ermittlung der Kenngröße erreicht werden. Das manuelle Abmessen, Berechnen und Notieren durch den Arbeiter entfällt. Dadurch kann die mindestens eine Kenngröße schneller, objektiver und genauer ermittelt werden. Bevorzugt ist die optische Erfassungseinheit eine Erfassungseinheit, die auch unter schlechten Lichtbedingungen oder ohne externe Lichtquelle arbeitet.

In vorteilhafter Ausführungsvariante wird eine Information über die Lage der mindestens einen optischen Erfassungseinheit zur Skalierung des optisch erfassten Parameters bei der automatisierten Bestimmung der mindestens einen Kenngröße genutzt. Insbesondere weist die Platte mindestens ein Mittel zur Skalierung auf. Das Mittel zur Skalierung kann insbesondere ein Maßstab oder dergleichen sein. Das Mittel zur Skalierung wird vorzugsweise optisch erfasst und zur Bestimmung der Lage der mindestens einen optischen Erfassungseinheit genutzt. Das Mittel zur Skalierung ist insbesondere so gestaltet, dass eine eindeutige Ermittlung von Position und Ausrichtung der optischen Erfassungseinheit möglich ist. Dadurch, dass die Platte das Mittel zur Skalierung aufweist, muss der Bediener lediglich die Platte mit der optischen Erfassungseinheit abbilden. Die optische Erfassungseinheit kann beispielsweise eine Kamera, insbesondere die Kamera in einem Smartphone oder dergleichen, sein. Zur Erfassung des mindestens einen Parameters muss der Arbeiter die Kamera lediglich grob positioniert über die Platte halten und die Platte fotografieren. Aus dem Bild wird die mindestens eine Kenngröße automatisiert bestimmt. Dadurch ist das Verfahren auf einfache Weise ohne spezielle Einrichtungen auf der Baustelle durchführbar. In alternativer Ausführung umfasst die optische Erfassungseinheit mindestens einen Laser. Unter dem Begriff "Laser" wird vorliegend dabei eine Laseranordnung aus Laserlichtquelle und Empfänger für das reflektierte Laserlicht verstanden. Insbesondere ist das Verfahren mittels einer auf einer Rechnereinheit wie beispielsweise einem Smartphone, einem Laptop oder dergleichen gespeicherten Anwendung ausführbar.

In alternativer Gestaltung kann vorgesehen sein, dass die mindestens eine optische Erfassungseinheit in einem definierten Abstand und in definierter Ausrichtung zu der Platte angeordnet wird. In diesem Fall können Mittel zur Skalierung auf der Platte entfallen oder zur zusätzlichen Plausibilitätsprüfung eingesetzt werden.

Vorzugsweise wird als Kenngröße das Ausbreitmaß des Frischbetons ermittelt. Zur Ermittlung des Ausbreitmaßes wird üblicherweise der Durchmesser der Probenmenge des Frischbetons in zwei Richtungen, vorzugsweise parallel zu den Kanten der Platte gemessen. Als Ausbreitmaß wird der Mittelwert der beiden Durchmesser angegeben. Insbesondere bei sehr unregelmäßiger Außenkontur der Probenmenge kann dies Schwierigkeiten bereiten oder zu falschen Ergebnissen führen. Erfindungsgemäß ist insbesondere vorgesehen, als Parameter zur Ermittlung des Ausbreitmaßes die Fläche des Frischbetons auf der Platte zu ermitteln. Die Fläche der Probenmenge bietet ein deutlich genaueres Maß als zwei Durchmesser der Probenmenge. Aus der Fläche der Probenmenge kann der Durchmesser eines Kreises mit gleichem Flächeninhalt berechnet und der errechnete Durchmesser als Ausbreitmaß gewertet werden. Alternativ oder ergänzend kann als Parameter mindestens ein Durchmesser des Frischbetons auf der Platte ermittelt werden. Vorzugsweise werden die nach der Norm vorgesehenen beiden parallel zu den Außenkanten der Platte gemessenen Durchmesser ermittelt und der Mittelwert als Ausbreitmaß gespeichert. Um besonders nachvollziehbare und aussagekräftige Werte zu erreichen, kann auch vorgesehen sein, sowohl die Fläche als auch mindestens einen Durchmesser des Frischbetons für das Ausbreitmaß zu ermitteln und abzuspeichern.

Besonders bevorzugt wird das Verfahren zur Ermittlung des visuellen Stabilitätsindex als Kenngröße eingesetzt. Der visuelle Stabilitätsindex wird bisher durch bloße Inaugenscheinnahme der Probenmenge durch die zuständige Person ermittelt. Der ermittelte Stabilitätsindex ist dadurch stark von der subjektiven Wahrnehmung der einzelnen Person abhängig. Vergleichbare Ergebnisse können nicht erzielt werden. Wird der visuelle Stabilitätsindex dagegen automatisiert bestimmt, so können festgelegte Kriterien angewendet werden, so dass sich wiederholbare und nachvollziehbare Ergebnisse ohne subjektiven Einfluss erzielen lassen. Zur Ermittlung des visuellen Stabilitätsindex wird als Parameter vorzugsweise die Größe des Bereichs, in dem Wasser aus dem Frischbeton ausblutet, ermittelt. Dieser Bereich erscheint üblicherweise als Kreisringfläche um den eigentlichen Probenkörper. Zur Ermittlung der Größe des Bereichs, in dem Wasser aus dem Frischbeton ausblutet, kann die Fläche des Bereichs, die Breite des Bereichs oder die Differenz des Außendurchmessers der gesamten Probenmenge zu der Probenmenge abzüglich des äußeren Bereichs, in dem sich ausgeblutetes Wasser angesammelt hat, ermittelt werden. Alternativ oder zusätzlich kann als Parameter zur Ermittlung des visuellen Stabilitätsindex die Größe des Bereichs in der Mitte der Probenmenge, in dem sich Zuschlag anhäuft und/oder Wasser ausblutet, ermittelt werden. Diese Bereiche lassen sich durch Bilderkennung identifizieren, und ihre Größe lässt sich dann als Parameter bestimmen, und zwar als Fläche oder näherungsweise über einen oder mehrere Durchmesser.

Alternativ oder zusätzlich kann als Parameter ein Höhenprofil des Frischbetons ermittelt werden. Aus dem Höhenprofil des Frischbetons kann als Kenngröße insbesondere eine Abschätzung für das Größtkorn des Frischbetons ermittelt werden. Es kann auch vorgesehen sein, eine Abschätzung für das Größtkorn des Frischbetons aus der optischen Abbildung der Probenmenge zu ermitteln, beispielsweise durch entsprechende Bilderkennungssoftware. Über die Abschätzung des Größtkorns des Frischbetons kann auf einfache Weise überprüft werden, ob die verwendeten Zuschläge den Spezifikationen des geprüften Frischbetons entsprechen.

Als weiterer Parameter kann die maximale Probenhöhe der Probenmenge des Frischbetons ermittelt werden. Die maximale Probenhöhe der Probenmenge gibt Aufschlüsse über die Konsistenz des Frischbetons. Mit der maximalen Probenhöhe kann neben dem Ausbreitmaß eine weitere Kenngröße für die Zähigkeit des Frischbetons angegeben werden.

Bevorzugt wird der Frischbeton mehrmals in zeitlichem Abstand optisch erfasst. Der zeitliche Abstand kann dabei fest vorgegeben sein oder erfasst und für die Auswertung herangezogen werden. Aus mehreren optischen Erfassungen in zeitlichem Abstand können Veränderungen der Parameter über die Zeit ermittelt werden. Aus den in zeitlichem Abstand erfassten Parametern wird vorzugsweise eine Ausbreitgeschwindigkeit des Frischbetons als Kenngröße ermittelt.

Alternativ oder zusätzlich kann vorgesehen sein, dass die Temperatur des Frischbetons erfasst und gespeichert wird. Die Temperatur des Frischbetons kann vorteilhaft mittels eines digitalen Thermometers oder eines Laserthermometers erfasst werden. Auch die Temperatur der Umgebung kann erfasst und gespeichert werden. Insbesondere wird überprüft, ob die Temperatur des Frischbetons und die Temperatur der Umgebung in den gewünschten Bereichen liegen. Die Temperatur des Frischbetons und/oder die Temperatur der Umgebung können dabei auch als Temperaturprofil über eine vorgegebene Zeit oder über mehrere Temperaturwerte, die in zeitlichem Abstand zueinander erfasst werden, ermittelt und abgespeichert werden.

Eine Anordnung zur Ermittlung mindestens einer Kenngröße von Frischbeton umfasst vorzugsweise eine Platte, mindestens eine optische Erfassungseinheit sowie einen Datenspeicher. Die Platte ist dabei vorzugsweise eine Platte, wie sie für herkömmliche Ausbreitversuche von Frischbeton verwendet wird. Die mindestens eine optische Erfassungseinheit ist bevorzugt eine Kamera oder ein Laser. Kameras sind einfach und günstig verfügbar, so dass sich insgesamt eine günstige Anordnung ergibt. Insbesondere kann eine ohnehin vorhandene Kamera, beispielsweise die Kamera eines Smartphones des Bedieners, zum Einsatz kommen. Bei Einsatz eines Lasers wird vorzugsweise ein Höhenprofil der Probenmenge ermittelt. Ein Laser wird durch Reflexionen, wie sie beispielsweise bei Verwendung einer Kamera durch ausblutendes Wasser entstehen können, nicht gestört, so dass die Ergebnisse bei Einsatz eines Lasers sehr zuverlässig sind.

Vorzugsweise umfasst die Anordnung einen Temperaturfühler. Es können ein oder mehrere Temperaturfühler zur Erfassung der Temperatur der Umgebung und/oder zur Erfassung der Temperatur des Betons vorgesehen sein. Der Temperaturfühler kann beispielsweise ein in dem Frischbeton anzuordnender Temperatursensor oder ein berührungslos arbeitendes Laserthermometer sein. Die optische Erfassungseinheit ist vorzugsweise höhenverstellbar und/oder schwenkbar gegenüber der Platte gehalten. Alternativ oder zusätzlich ist vorgesehen, dass die Platte Mittel zur Skalierung aufweist. Die Mittel zur Skalierung können vorzugsweise einen Maßstab umfassen.

Das Verfahren sieht vorzugsweise ergänzend vor, die Charge der Probenmenge zu ermitteln und zu speichern. Dies kann beispielsweise mit Hilfe eines vom Bediener einzulesenden QR-Codes erfolgen. Eine einfache Handhabung der Anordnung wird erreicht, wenn die Erfassungseinheit die Chargenbezeichnung aus einem vom Bediener erstellten Foto, beispielsweise von einem Lieferschein oder dergleichen ermittelt. Alternativ kann ein solches Foto zur Dokumentation der Chargenbezeichnung in dem Datenspeicher abgespeichert werden.

Ausführungsbeispiele der Erfindung werden im Folgenden anhand der Zeichnung erläutert. Es zeigen:
- Fig. 1: eine schematische Seitenansicht einer Anordnung zur Ermittlung mindestens einer Kenngröße von Frischbeton,
- Fig. 2: eine schematische Draufsicht auf die Platte der Anordnung aus Fig. 1,
- Fig. 3: eine schematische Darstellung eines Höhenprofils einer Probenmenge,
- Fig. 4 und Fig. 5: schematische Darstellungen von dreidimensionalen Diagrammen von Probekörpern.

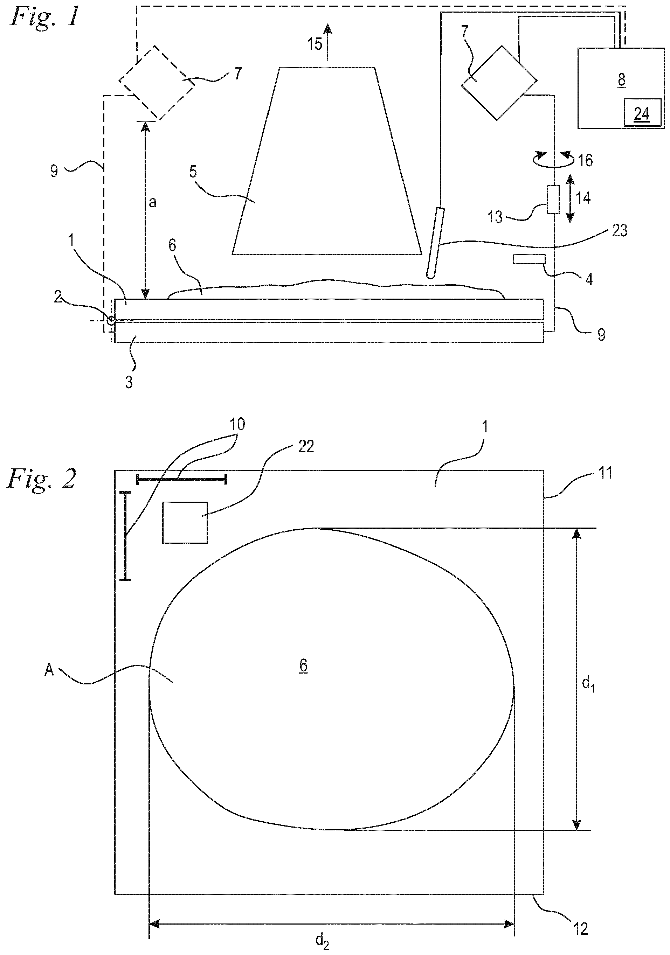

Fig. 1 zeigt schematisch eine Platte 1, auf der eine Probenmenge aus Frischbeton 6 angeordnet ist. Die Platte 1 ist über ein Gelenk 2 schwenkbar an einer Grundplatte 3 gelagert. Oberhalb der Platte 1 ist ein Anschlag 4 vorgesehen. Zum Ausbringen des Frischbetons 6 ist ein schematisch dargestellter Ausgießkonus 5 vorgesehen. Der Ausgießkonus 5 wird zur Herstellung einer Probenmenge von Frischbeton 6 auf die Platte 1 gestellt, mit Frischbeton 6 gefüllt und dann in Richtung des Pfeils 15 von der Platte 1 abgehoben. Die Platte 1 wird dann in vorgegebener Weise mehrfach bis zum Anschlag 4 nach oben angehoben und fallen gelassen. Durch diese Bewegung fließt der Frischbeton 6 in die Breite. Die Art und Weise, wie die Probenmenge vom Bediener herzustellen ist, entspricht vorzugsweise der normierten Vorgehensweise bei der Ermittlung des Ausbreitmaßes.

Die Anordnung weist mindestens eine optische Erfassungseinheit 7, vorzugsweise eine Kamera oder einen Laser auf, die oberhalb der Platte 1 mittels Halterungen 9 montiert ist. Im Ausführungsbeispiel ist eine optische Erfassungseinheit 7 mit durchgehender Linie dargestellt und eine weitere, optionale optische Erfassungseinheit 7 mit gestrichelter Linie dargestellt. Die mindestens eine optische Erfassungseinheit 7 ist mit einer Auswerteeinheit 8 verbunden, die im Ausführungsbeispiel einen Datenspeicher 24 umfasst. Der Datenspeicher 24 kann auch separat von der Auswerteeinheit 8 ausgebildet sein. Die Verbindung zwischen optischer Erfassungseinheit 7 kann in jeder üblichen Weise, beispielsweise kabelgebunden oder kabellos erfolgen.

Die Halterung 9 weist im Ausführungsbeispiel eine Verstelleinrichtung 13 auf, die eine Höhenverstellung in Richtung eines Doppelpfeils 14 sowie eine Schwenkbewegung in Richtung des Doppelpfeils 16 erlaubt. Dadurch kann die optische Erfassungseinheit 7 in geeigneter Weise ausgerichtet werden. Alternativ kann auch vorgesehen sein, dass die optische Erfassungseinheit 7 in vorgegebenem Abstand a oberhalb der Platte 1 montiert ist, wie in Fig. 1 für die mit gestrichelter Linie dargestellte, optionale optische Erfassungseinheit 7 angedeutet.

Wie Fig. 2 zeigt, ist die Platte 1 rechteckig, vorzugsweise quadratisch, und weist zwei Längsseiten 11 und 12 auf. Die Platte 1 kann zusätzlich einen Maßstab 10 aufweisen. Im Ausführungsbeispiel sind zwei rechtwinklig zueinander angeordnete Maßstäbe 10 vorgesehen. Ergänzend kann an der Platte 1 ein Feld 22 zur Aufnahme von Informationen über den Frischbeton 6, beispielsweise eine Chargennummer, insbesondere in Form eines QR-Codes, vorgesehen sein.

Zur Ermittlung des Ausbreitmaßes kann über die optischen Erfassungseinheiten 7, insbesondere über Kameras, ein Bild des Frischbetons 6 aufgenommen werden. Sind die optischen Erfassungseinheiten 7 fest installiert, so kennt die Auswerteeinheit 8 Lage und Ausrichtung der mindestens einen optischen Erfassungseinheit 7 zur Platte 1. Wird die optische Erfassungseinheit 7 vom Bediener frei über der Platte 1 positioniert, so ermittelt die Auswerteeinheit 8 aus dem mindestens einen Maßstab 10 die Lage und Ausrichtung der optischen Erfassungseinheit 7. Aus diesen Angaben kann eine Skalierung und ggf. die Verzerrung für das aufgenommene Bild errechnet werden. Zur Ermittlung des Ausbreitmaßes kann vorgesehen sein, einen ersten Durchmesser d₁ parallel zur ersten Längsseite 11 sowie einen zweiten Durchmesser d₂ parallel zur zweiten Längsseite 12 zu messen. Die Durchmesser d₁ und d₂ sind dabei die größten Durchmesser in der jeweiligen Richtung. Das Ausbreitmaß kann als Mittelwert der beiden Durchmesser d₁ und d₂ angegeben werden. Bevorzugt ist vorgesehen, dass anstatt oder zusätzlich zu den Durchmessern d₁ und d₂ eine Fläche A des Frischbetons 6 ermittelt wird. Aus der Fläche A wird vorzugsweise der Durchmesser eines Kreises mit gleichem Flächeninhalt A errechnet und der Durchmesser dieses Kreises wird als Ausbreitmaß angegeben. Dadurch lässt sich das Ausbreitmaß deutlich genauer angeben als durch Angabe des Mittelwerts von lediglich zwei Durchmessern.

In Fig. 1 ist ergänzend ein Temperaturfühler 23 dargestellt, der ebenfalls einen Teil der Anordnung bildet. Vorzugsweise wird die Temperatur der Umgebung und/oder die Temperatur des Frischbetons 6 ermittelt und in der Auswerteeinheit 8 ausgewertet. Hierbei wird insbesondere ausgewertet, ob die gemessenen Temperaturen im gewünschten Bereich liegen. Der oder die Temperaturwerte werden vorzugsweise im Datenspeicher 24 abgespeichert. Die Temperatur kann auch über einen längeren Zeitraum oder mehrfach mit zeitlichem Abstand ermittelt und ausgewertet werden. Die Temperatur des Frischbetons 6 kann beispielsweise ein Temperatursensor oder ein Laserthermometer sein. Es kann vorgesehen, dass der Temperaturfühler 23 mit der Auswerteeinheit 8 Daten austauscht. Alternativ kann der Temperaturfühler 23 eine nicht dargestellte Temperaturanzeige aufweisen, die von der optischen Erfassungseinheit 7 abgebildet und mittels Bilderkennung, insbesondere in der Auswerteeinheit 8, ausgewertet wird.

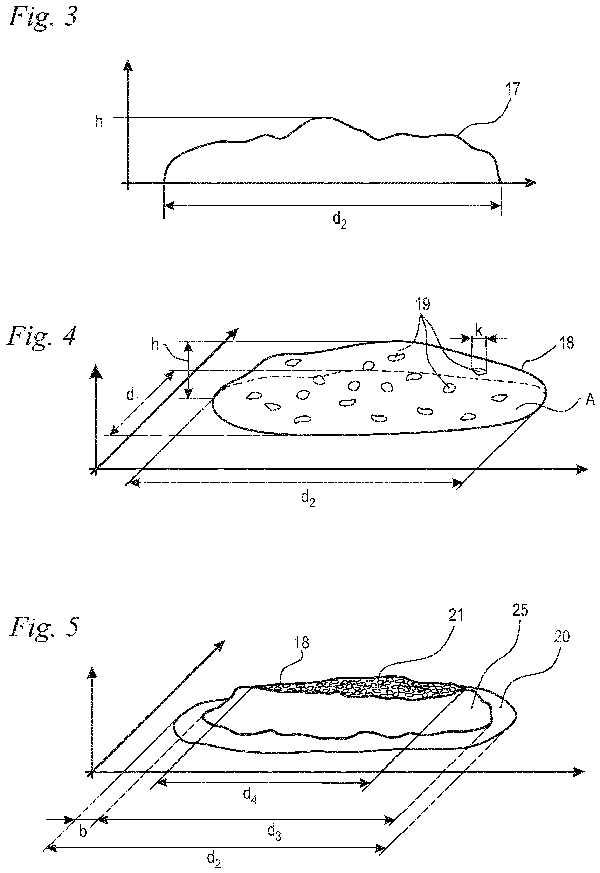

Die optische Erfassungseinheit 7, beispielsweise ein Laser oder eine Kamera, kann das in Fig. 3 schematisch dargestellte Höhenprofil 17 als Parameter des Frischbetons 6 erfassen. Aus dem Höhenprofil 17 können die maximale Probenhöhe h der Probenmenge, ein Durchmesser, im Ausführungsbeispiel der Durchmesser d₂, sowie das Größtkorn des Frischbetons ermittelt werden. Aus dem Höhenprofil kann außerdem der visuelle Stabilitätsindex ermittelt werden, wenn die Größe des Bereichs, in dem Wasser aus dem Frischbeton ausblutet, und/oder die Größe des Bereichs, in dem sich Zuschlag ansammelt, aus dem Höhenprofil 17 ausgelesen wird. Diese Bereiche werden im Folgenden noch näher zu Fig. 5 erläutert.

Alternativ oder zusätzlich kann die in Fig. 4 schematisch dargestellte dreidimensionale Abbildung 18 der Probenmenge von Frischbeton 6 erstellt werden, insbesondere, wenn als optische Erfassungseinheit 7 mindestens ein Laser zum Einsatz kommt. Bevorzugt sind mindestens zwei Laser vorgesehen. Aus der dreidimensionalen Abbildung 18 können sowohl die Durchmesser d₁ und d₂ und die Probenhöhe h der Probenmenge ermittelt werden als auch die Fläche A, die die Probenmenge auf der Platte 1 bedeckt. Ergänzend kann die Korngröße k von Körnern 19 der Zuschlagstoffe ermittelt werden. Die Körner 19 sind in Fig. 4 schematisch dargestellt. Aus der Korngröße k kann das Größtkorn der Zuschlagstoffe im Frischbeton 6 ermittelt werden. Das ermittelte Größtkorn ist dabei bevorzugt eine Abschätzung für das Größtkorn.

Fig. 5 zeigt schematisch eine dreidimensionale Abbildung 18 einer weiteren Probenmenge von Frischbeton 6. Bei der in Fig. 5 dargestellten Probenmenge hat der Beton deutlich ausgeblutet, so dass sich um den eigentlichen, Zuschlagstoffe enthaltenden inneren Bereich 25 der Probenmenge ein Bereich 20 gebildet hat, in dem Wasser abgesondert wurde. Die Probenmenge besitzt außerdem einen inneren, oberen Bereich 21, in dem Zuschlagstoffe angehäuft sind. In dem inneren, oberen Bereich 21 kann alternativ oder ergänzend zur Anhäufung von Zuschlagstoffen Wasser abgesondert sein. Der Frischbeton 6 ist daher sehr instabil, und der visuelle Stabilitätsindex ist hoch. Zur wiederholbaren nachvollziehbaren Ermittlung des visuellen Stabilitätsindex ist vorgesehen, den zweiten Durchmesser d₂ der gesamten Probenmenge, einen dritten Durchmesser d₃ des inneren Bereichs 25 des Frischbetons 6 sowie vorzugsweise ergänzend einen vierten Durchmesser d₄ des Bereichs 21, in dem Zuschlag angehäuft ist und/oder Wasser ausgeblutet ist, zu ermitteln. Der zweite, dritte und vierte Durchmesser könnte dabei als Einzelwerte in bestimmter Richtung gemessen oder aus der exakt ermittelten Fläche als Durchmesser eines Kreises mit gleichem Flächeninhalt errechnet werden. Alternativ oder ergänzend kann die Breite b des Bereichs 20 gemessen werden. Die Breite b kann jedoch auch als halbe Differenz von zweitem Durchmesser d₂ und drittem Durchmesser d₃ errechnet werden. Zur Ermittlung des visuellen Stabilitätsindex wird vorzugsweise ermittelt, innerhalb welchen Grenzen die Durchmesser d₂, d₃ und d₄ liegen, und hieraus wird der visuelle Stabilitätsindex bestimmt. Alternativ können die Durchmesser d₂ und d₄ auf den Durchmesser d₃ bezogen ausgewertet werden. Dadurch kann eine objektive und nachvollziehbare Ermittlung des visuellen Stabilitätsindex erfolgen.

Auch weitere Parameter und Kenngrößen des Frischbetons können ermittelt werden. Alle oben genannten Parameter und Kenngrößen können einzeln oder beliebig kombiniert ermittelt und zu Charakterisierung des Frischbetons herangezogen werden.

## Patentansprüche

1. Verfahren zur Ermittlung mindestens einer Kenngröße von Frischbeton, wobei eine Probenmenge des Frischbetons (6) auf eine Platte (1) aufgebracht wird, wobei das Verfahren die folgenden Schritte umfasst:
- optische Erfassung mindestens eines Parameters des Frischbetons (6) mittels einer optischen Erfassungseinheit (7),
- automatisierte Bestimmung mindestens einer Kenngröße des Frischbetons (6) aus dem mindestens einen Parameter und
- Speicherung der mindestens einen Kenngröße in einem Datenspeicher (24).

2. Verfahren nach Anspruch 1,
**dadurch gekennzeichnet, dass** eine Information über die Lage der mindestens einen optischen Erfassungseinheit (7) zur Skalierung des optisch erfassten Parameters bei der automatisierten Bestimmung der mindestens einen Kenngröße genutzt wird.

3. Verfahren nach Anspruch 2,
**dadurch gekennzeichnet, dass** die Platte (1) mindestens ein Mittel zur Skalierung aufweist, wobei das Mittel zur Skalierung optisch erfasst und zur Bestimmung der Lage der mindestens einen optischen Erfassungseinheit (7) genutzt wird.

4. Verfahren nach Anspruch 2,
**dadurch gekennzeichnet, dass** die mindestens eine optische Erfassungseinheit (7) in einem definierten Abstand (a) und in definierter Ausrichtung zu der Platte (1) angeordnet wird.

5. Verfahren nach einem der Ansprüche 1 bis 4,
**dadurch gekennzeichnet, dass** als Kenngröße das Ausbreitmaß des Frischbetons (6) ermittelt wird, wobei als Parameter zur Ermittlung des Ausbreitmaßes insbesondere die Fläche (A) des Frischbetons (6) auf der Platte (1) und/oder mindestens ein Durchmesser (d₁, d₂) des Frischbetons (6) auf der Platte (1) ermittelt wird.

6. Verfahren nach einem der Ansprüche 1 bis 5,
**dadurch gekennzeichnet, dass** als Kenngröße der visuelle Stabilitätsindex des Frischbetons (6) ermittelt wird, wobei als Parameter zur Ermittlung des visuellen Stabilitätsindex insbesondere die Größe des Bereichs (20), in dem Wasser aus dem Frischbeton (6) ausblutet und/oder die Größe des Bereichs (21), in dem sich Zuschlag anhäuft, ermittelt wird.

7. Verfahren nach einem der Ansprüche 1 bis 6,
**dadurch gekennzeichnet, dass** als Parameter ein Höhenprofil (17) des Frischbetons (6) ermittelt wird und/oder, dass als Parameter die maximale Probenhöhe (h) der Probenmenge des Frischbetons (6) ermittelt wird.

8. Verfahren nach einem der Ansprüche 1 bis 7,
**dadurch gekennzeichnet, dass** eine Abschätzung für das Größtkorn des Frischbetons (6) als Kenngröße ermittelt wird.

9. Verfahren nach einem der Ansprüche 1 bis 8,
**dadurch gekennzeichnet, dass** der Frischbeton (6) mehrmals in zeitlichem Abstand optisch erfasst wird, wobei aus den in zeitlichem Abstand erfassten Parametern insbesondere eine Ausbreitgeschwindigkeit des Frischbetons (6) als Kenngröße ermittelt wird.

10. Verfahren nach einem der Ansprüche 1 bis 9,
**dadurch gekennzeichnet, dass** die Temperatur des Frischbetons (6) erfasst und gespeichert wird.

11. Anordnung zur Ermittlung mindestens einer Kenngröße von Frischbeton, insbesondere nach einem der Ansprüche 1 bis 10, wobei die Anordnung eine Platte (1), mindestens eine optische Erfassungseinheit (7) sowie einen Datenspeicher (24) umfasst.

12. Anordnung nach Anspruch 11,
**dadurch gekennzeichnet, dass** mindestens eine optische Erfassungseinheit (7) eine Kamera oder ein Laser ist.

13. Anordnung nach Anspruch 11 oder 12,
**dadurch gekennzeichnet, dass** die Anordnung einen Temperaturfühler (23) umfasst.

14. Anordnung nach einem der Ansprüche 11 bis 13,
**dadurch gekennzeichnet, dass** die optische Erfassungseinheit (7) höhenverstellbar und/oder schwenkbar gegenüber der Platte (1) gehalten ist.

15. Anordnung nach einem der Ansprüche 11 bis 14,
**dadurch gekennzeichnet, dass** die Platte (1) Mittel zur Skalierung aufweist.
